# EUROPEAN PATENT APPLICATION

(11) **EP 4 388 988 A1**
(43) Date of publication of application: **26.06.2024**
(21) Application number: 22906300.3
(22) Date of filing: 05.12.2022
(51) Int. Cl.: A61B 5/01, A61B 5/33

(54) **BODY TEMPERATURE MEASUREMENT METHOD, AND ELECTRONIC DEVICE**

(30) Priority: 15.12.2021 CN 202111538759
(71) Applicant: Huawei Technologies Co., Ltd., Shenzhen, Guangdong 518129 (CN)
(72) Inventor: SHENG, Rong, Shenzhen, Guangdong 518129 (CN); HE, Zhijian, Shenzhen, Guangdong 518129 (CN); WANG, Runsen, Shenzhen, Guangdong 518129 (CN); WANG, Lu, Shenzhen, Guangdong 518129 (CN); HAN, Yujia, Shenzhen, Guangdong 518129 (CN)
(74) Representative: Pfenning, Meinig & Partner mbB
(86) International application number: PCT/CN2022/136596
(87) International publication number: WO 2023/109550

(57) **Abstract**

A body temperature measurement method and an electronic device are provided, to improve accuracy of a body temperature obtained through measurement. The method includes: obtaining a target parameter, where the target parameter includes motion data of a user and a current heart rate value of the user, the motion data is used to determine a current motion status of the user; determining a body temperature of the user based on the current motion status of the user and the current heart rate value of the user; and reminding the user of the body temperature.

## Description

This application claims priority to Chinese Patent Application No. 202111538759.1, filed with the China National Intellectual Property Administration on December 15, 2021 and entitled "BODY TEMPERATURE MEASUREMENT METHOD AND ELECTRONIC DEVICE", which is incorporated herein by reference in its entirety.

### TECHNICAL FIELD

This application relates to the field of electronic device technologies, and in particular, to a body temperature measurement method and an electronic device.

### BACKGROUND

In daily life, a user sometimes has a requirement for measuring a body temperature. Therefore, various types of body temperature measurement electronic devices (such as a smart watch, a smart band, and a handhold thermometer) emerge in the market to measure the body temperature of the user. For example, the electronic device obtains the body temperature of the user by detecting a temperature of partial skin (for example, skin of a part on which a wearable device having a temperature measurement function is worn) of a human body, or the electronic device estimates the body temperature by detecting a heart rate. However, accuracy of body temperatures obtained through measurement by using these solutions is low.

### SUMMARY

This application provides a body temperature measurement method and an electronic device, to improve accuracy of a body temperature obtained through measurement.

To achieve the foregoing objective, the following technical solutions are used in this application.

According to a first aspect, this application provides a body temperature measurement method, applied to an electronic device. The method includes: obtaining a target parameter, where the target parameter includes motion data of a user and a current heart rate value of the user, and the motion data is used to determine a current motion status of the user; determining a body temperature of the user based on the current motion status of the user and the current heart rate value of the user; and reminding the user of the body temperature.

Based on the foregoing technical solution, the electronic device can estimate the body temperature of the user based on a plurality of obtained physiological parameters such as the current motion status of the user and the current heart rate value of the user. Impact of a motion status of the user on a heart rate of the user and further on an estimated body temperature is considered, so that accuracy of the estimated body temperature can be improved.

In a possible implementation, the determining a body temperature of the user based on the current motion status of the user and the current heart rate value of the user includes: determining a target heart rate baseline based on the current motion status of the user, where the target heart rate baseline indicates a heart rate status of the user in the current motion status; and determining the body temperature of the user based on the current heart rate value of the user and the target heart rate baseline.

Based on this implementation, each user may have a different heart rate characteristic, that is, may have a different heart rate baseline. In addition, a same user may also have different heart rate baselines in different motion statuses. Therefore, the electronic device first determines a corresponding heart rate baseline based on the current motion status of the user, and then estimates a body temperature of the user based on the determined heart rate baseline and the current heart rate value of the user, to enable the estimated body temperature to be more accurate.

In a possible implementation, before the determining a target heart rate baseline based on the current motion status of the user, the method further includes: obtaining account information of the user; and obtaining a mapping relationship between different motion statuses and heart rate baselines of the user based on the account information. The determining a target heart rate baseline based on the current motion status of the user includes: determining the target heart rate baseline based on the current motion status of the user and the mapping relationship.

Based on this implementation, each user has account information of the user. When measuring a body temperature of a user, the electronic device may obtain, based on account information of the user, a mapping relationship between different motion statuses corresponding to the user and heart rate baselines, and further determines a corresponding heart rate baseline based on the mapping relationship and a current motion status of the user, so that accuracy of the obtained heart rate baseline can be improved. Then, the electronic device estimates a body temperature of the user based on the determined heart rate baseline and the current heart rate value of the user, to enable the estimated body temperature to be more accurate.

In a possible implementation, before the determining a target heart rate baseline based on the current motion status of the user, the method further includes: if no mapping relationship between different motion statuses and heart rate baselines of the user is obtained, reminding the user to establish the mapping relationship.

Based on this implementation, when the body temperature of the user is measured, if no mapping relationship between different motion statuses and heart rate baselines that correspond to the user is obtained, the user may be reminded to establish the mapping relationship, so that the user can establish the mapping relationship based on a prompt, and efficiency of human-computer interaction can be improved. In addition, when body temperature measurement is subsequently performed on the user, accuracy of a body temperature that is of the user and that is obtained through measurement is improved.

In a possible implementation, if no mapping relationship between different motion statuses and heart rate baselines of the user is obtained, the method further includes: obtaining heart rate values of the user in the different motion statuses; and establishing the mapping relationship based on the different motion statuses and the heart rate values.

Based on this implementation, each user may have a different heart rate characteristic, that is, may have a different heart rate baseline. In addition, a same user may also have different heart rate baselines in different motion statuses. Therefore, if no mapping relationship between different motion statuses and heart rate baselines corresponding to the user is obtained, the electronic device obtains the heart rate values of the user in the different motion statuses, and then establishes the mapping relationship based on the different motion statuses and the obtained heart rate values. In other words, the estimated body temperature is enabled to be more accurate in a manner of establishing a heart rate baseline.

In a possible implementation, the obtained heart rate values of the user in the different motion statuses meet a requirement for a confidence.

Based on this implementation, in a process in which the electronic device establishes the heart rate baseline, the obtained heart rate values of the user in the different motion statuses meet a requirement for a confidence. In other words, an obtained heart rate value can be used to establish the heart rate baseline only when the heart rate value meets the requirement for the confidence; and an obtained heart rate cannot be used to establish the heart rate baseline when the heart rate value does not meet the requirement for the confidence. In this way, accuracy of the established heart rate baseline can be improved. Further, when the heart rate baseline is subsequently used to estimate a body temperature, accuracy of the body temperature obtained through measurement is improved.

In a possible implementation, the confidence is determined based on one or more factors in quality of a signal used to measure a heart rate of the user and the current motion status of the user.

In a possible implementation, the target parameter further includes one or more of a skin temperature of a target part of the user and a current ambient temperature. The method further includes: determining the body temperature of the user based on the current motion status of the user, the current heart rate value of the user, the skin temperature of the target part of the user, and/or the current ambient temperature.

Based on this implementation, the electronic device can estimate the body temperature of the user based on a plurality of obtained parameters such as the current motion status of the user, the current heart rate value of the user, the skin temperature of the target part of the user, and/or the current ambient temperature, so that accuracy of the estimated body temperature can be improved.

In a possible implementation, the different motion statuses include a sleep state and a non-sleep state.

In a possible implementation, the non-sleep state includes a low activity amount state, a medium activity amount state, and a high activity amount state. The low activity amount state is a state in which an activity amount of the user is less than a first threshold, the medium activity amount state is a state in which an activity amount of the user is greater than or equal to the first threshold and less than or equal to a second threshold, and the high activity amount state is a state in which an activity amount of the user is greater than the second threshold.

In a possible implementation, the different motion statuses include one or more of the following: a slow-walking state, a fast-walking state, a jogging state, a fast-running state, a riding state, and a rope skipping state.

In a possible implementation, the reminding the user of the body temperature includes: displaying the body temperature; and/or broadcasting the body temperature by voice.

According to a second aspect, this application provides an electronic device, including a processor and a memory. The memory is coupled to the processor. The memory is configured to store computer program code, and the computer program code includes computer instructions. When the processor reads the computer instructions from the memory, the electronic device is enabled to perform the method according to any one of the first aspect and the possible implementations of the first aspect.

According to a third aspect, this application provides an electronic device. The electronic device has a function of implementing the method according to any one of the first aspect and the possible implementations of the first aspect. The function may be implemented by hardware, or may be implemented by hardware executing corresponding software. The hardware or the software includes one or more modules corresponding to the function.

According to a fourth aspect, this application provides a computer-readable storage medium. The computer-readable storage medium stores a computer program or instructions. When the computer program or the instructions is/are run on a computer, the computer is enabled to perform the method according to any one of the first aspect and the possible implementations of the first aspect.

According to a fifth aspect, this application provides a computer program product, where the computer program product includes a computer program or instructions. When the computer program or the instructions is/are run on a computer, the computer is enabled to perform the method according to any one of the first aspect and the possible implementations of the first aspect.

According to a sixth aspect, an embodiment of this application provides a chip system, including at least one processor and at least one interface circuit. The at least one interface circuit is configured to: perform a transceiver function, and send instructions to the at least one processor. When the at least one processor executes the instructions, the at least one processor performs the method according to any one of the first aspect and the possible implementations of the first aspect.

It should be noted that, for technical effects brought by any possible implementation in the second to the sixth aspects, refer to technical effects brought by corresponding implementations in the first aspect. Details are not described herein again.

### BRIEF DESCRIPTION OF DRAWINGS

FIG. 1 is a schematic diagram of results of estimating a body temperature of a user by using an existing solution according to an embodiment of this application;
FIG. 2 is a schematic diagram of a structure of an electronic device according to an embodiment of this application;
FIG. 3 is a schematic diagram of a hardware structure of an electronic device according to an embodiment of this application;
FIG. 4 is a block diagram of a software structure of an electronic device according to an embodiment of this application;
FIG. 5a is a schematic diagram of a related interface for body temperature measurement according to an embodiment of this application;
FIG. 5b is a schematic diagram of another related interface for body temperature measurement according to an embodiment of this application;
FIG. 5c is a schematic diagram of another related interface for body temperature measurement according to an embodiment of this application;
FIG. 6A to FIG. 6D are a schematic diagram of a related interface for reminding a user according to an embodiment of this application;
FIG. 7 is a schematic flowchart of a body temperature measurement method according to an embodiment of this application;
FIG. 8 is a schematic flowchart of a method for establishing a mapping relationship between different motion statuses and heart rate baselines of a user according to an embodiment of this application;
FIG. 9a is a schematic diagram of an interface for enabling a continuous heart rate monitoring function of an electronic device according to an embodiment of this application;
FIG. 9b is a schematic diagram of another related interface for enabling a continuous heart rate monitoring function of an electronic device according to an embodiment of this application;
FIG. 9c is a schematic diagram of another related interface for enabling a continuous heart rate monitoring function of an electronic device according to an embodiment of this application;
FIG. 9d is a schematic diagram of another related interface for enabling a continuous heart rate monitoring function of an electronic device according to an embodiment of this application;
FIG. 9e-1 to FIG. 9e-4 are a schematic diagram of another related interface for enabling a continuous heart rate monitoring function of an electronic device according to an embodiment of this application;

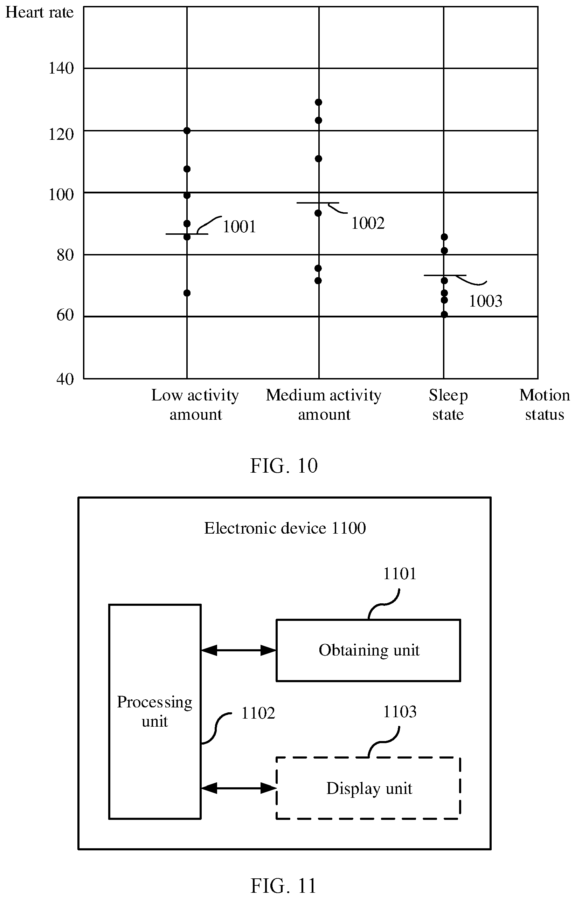

FIG. 10 is a schematic diagram of a heart rate baseline according to an embodiment of this application;
FIG. 11 is a schematic diagram of a structure of an electronic device according to an embodiment of this application; and

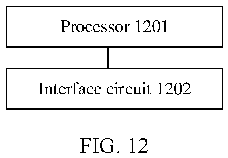

FIG. 12 is a schematic diagram of a structure of a chip system according to an embodiment of this application.

### DESCRIPTION OF EMBODIMENTS

The following describes, in detail with reference to the accompanying drawings, a body temperature measurement method and an electronic device that are provided in embodiments of this application.

The terms "including", "having", or any other variant thereof in descriptions of this application are intended to cover a non-exclusive inclusion. For example, a process, a method, a system, a product, or a device that includes a series of steps or units is not limited to the listed steps or units, but optionally further includes other unlisted steps or units, or optionally further includes another inherent step or unit of the process, the method, the product, or the device.

It should be noted that, in embodiments of this application, the word like "example" or "for example" is used to represent giving an example, an illustration, or a description. Any embodiment or design scheme described as an "example" or "for example" in embodiments of this application should not be explained as being more preferred or having more advantages than another embodiment or design scheme. Exactly, use of the word like "example" or "for example" is intended to present a related concept in a specific manner.

In the descriptions of this application, unless otherwise stated, "a plurality of" means two or more than two. A term "and/or" in this specification describes only an association relationship between associated objects and represents that three relationships may exist. For example, A and/or B may represent the following three cases: Only A exists, both A and B exist, and only B exists.

Currently, in some existing body temperature measurement solutions, a body temperature status of a human body may be reflected by directly measuring a temperature of partial skin (for example, skin of a part on which a wearable device having a temperature measurement function is worn) of the human body. However, because a skin temperature is greatly affected by an external environment, the skin temperature measured in this manner is not a real body temperature of the human body, and accuracy of the body temperature obtained through measurement in this solution is low.

In some other existing solutions, a body temperature of the human body is further estimated by measuring a heart rate of the human body. However, an algorithm model used in this solution is determined by collecting statistics on heart rate characteristics of most users. Some users' heart rate characteristics may be different from those of most users. For example, a heart rate may be higher or may be lower. Therefore, this solution is applicable only to a user whose heart rate characteristic meets an expectation of the algorithm model, that is, applicable only to some users. In addition, a motion status also affects a heart rate of a user. During and/or after motion of the user, the heart rate of the user may be higher. In this case, a body temperature of the user cannot be estimated according to this solution. Therefore, accuracy of a body temperature obtained through measurement by using this solution is low.

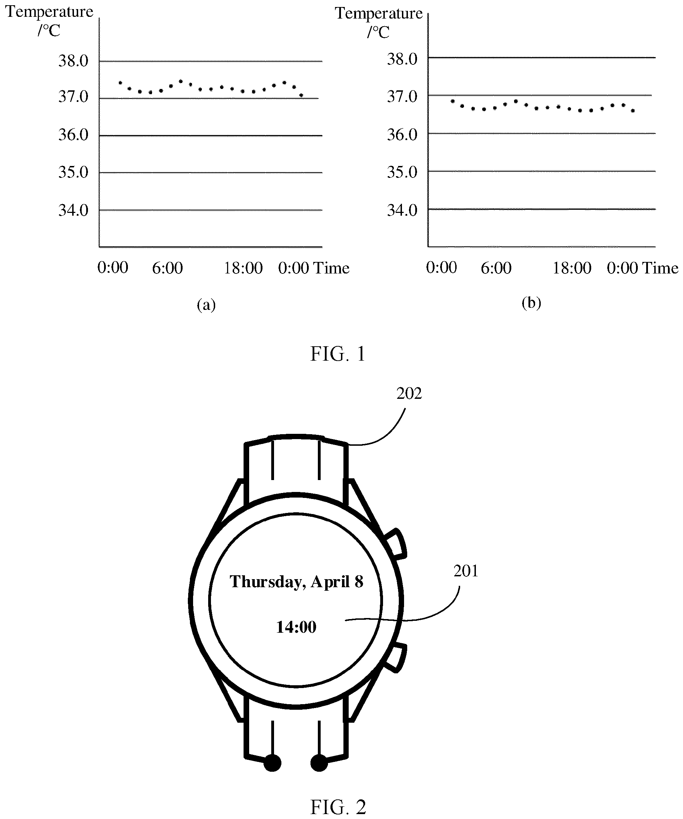

For example, as shown in FIG. 1, (a) in FIG. 1 and (b) in FIG. 1 respectively show body temperature data of a user 1 and body temperature data of a user 2, where the body temperature data of the user 1 and the body temperature data of the user 2 are obtained through estimation based on heart rates in an existing solution. A heart rate of the user 1 is higher than a heart rate of the user 2. Therefore, estimated body temperature values of the user 1 are higher. This may lead to a case in which a body temperature of the user 1 is actually normal, but a measured body temperature of the user 1 is higher than the normal body temperature. Consequently, a measurement result is inaccurate.

Therefore, to resolve the foregoing technical problem, an embodiment of this application provides a body temperature measurement method, including: An electronic device obtains a target parameter, where the target parameter includes motion data of a user, a current heart rate value of the user, and the like, and the motion data of the user is used to determine a current motion status of the user. If a mapping relationship between different motion statuses and heart rate baselines of the user is obtained, the electronic device determines a target heart rate baseline based on the mapping relationship and the current motion status of the user, where the target heart rate baseline indicates a heart rate status of the user in the current motion status. Then, the electronic device determines a body temperature of the user based on the current heart rate value of the user and the target heart rate baseline, and reminds the user of the body temperature. It may be understood that a heart rate baseline is a heart rate reference, and may be specifically a heart rate value, or may be a heart rate range. Each user may have a different heart rate characteristic, that is, may have a different heart rate baseline. In addition, a same user may also have different heart rate baselines in different motion statuses. Therefore, a corresponding heart rate baseline is first determined based on the current motion status of the user, and then a body temperature is estimated based on the determined heart rate baseline and the current heart rate value of the user, so that accuracy of the body temperature obtained through measurement can be improved.

The body temperature measurement method provided in this embodiment of this application may be applied to an electronic device 200, or may be applied to a system including the electronic device 200.

Optionally, the electronic device 200 may be a mobile phone, an artificial intelligence (artificial intelligence, AI) device, a wearable device, a vehicle-mounted device, a smart home device, and/or a smart city device. The wearable device includes but is not limited to a smart watch, a smart band, a smart anklet, a wireless headset, smart glasses, a smart helmet, a handhold thermometer, a thermometer, a forehead thermometer, an ear thermometer, or the like. A specific type of the electronic device 200 is not limited in embodiments of this application.

For example, the electronic device 200 is a wearable device. For example, FIG. 2 shows a schematic diagram of a structure of the electronic device 200. The electronic device 200 may be worn on a wrist of a user. The electronic device 200 includes a display 201 and a fixing band 202. The display 201 is configured to: display time, display a body temperature, and receive touch and tap operations of the user to display other related content. The fixing band 202 may be configured to fasten the electronic device 200 to the wrist of the user.

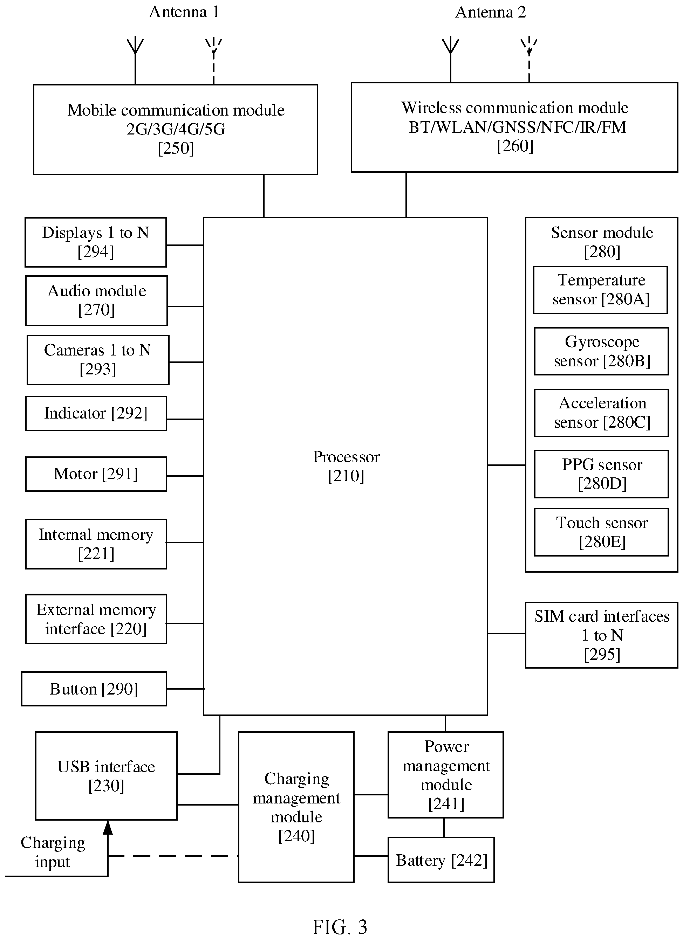

For example, FIG. 3 shows a schematic diagram of a hardware structure of the electronic device 200.

The electronic device 200 may include a processor 210, an external memory interface 220, an internal memory 221, a universal serial bus (universal serial bus, USB) interface 230, a charging management module 240, a power management module 241, a battery 242, an antenna 1, an antenna 2, a mobile communication module 250, a wireless communication module 260, an audio module 270, a sensor module 280, a button 290, a motor 291, an indicator 292, a camera 293, a display 294, a subscriber identification module (subscriber identification module, SIM) card interface 295, and the like. The sensor module 280 may include a temperature sensor 280A, a gyroscope sensor 280B, an acceleration sensor 280C, a photoplethysmograph (photoplethysmograph, PPG) sensor 280D, a touch sensor 280E, and the like.

It may be understood that the structure shown in this embodiment of the present invention does not constitute a specific limitation on the electronic device 200. In some other embodiments of this application, the electronic device 200 may include more or fewer components than those shown in the figure, or some components may be combined, or some components maybe split, or there may be different component arrangements. The components shown in the figure may be implemented by hardware, software, or a combination of software and hardware.

The processor 210 may include one or more processing units. For example, the processor 210 may include an application processor (application processor, AP), a modem processor, a graphics processing unit (graphics processing unit, GPU), an image signal processor (image signal processor, ISP), a controller, a video codec, a digital signal processor (digital signal processor, DSP), a baseband processor, and/or a neural-network processing unit (neural-network processing unit, NPU). Different processing units may be independent components, or may be integrated into one or more processors.

The controller may generate an operation control signal based on an instruction operation code and a time sequence signal, to complete control of instruction reading and instruction execution.

A memory may be further disposed in the processor 210, and is configured to store instructions and data. In some embodiments, the memory in the processor 210 is a cache. The memory may store instructions or data just used or cyclically used by the processor 210. If the processor 210 needs to use the instructions or the data again, the processor 210 may directly invoke the instructions or the data from the memory. This avoids repeated access and reduces waiting time of the processor 210, thereby improving system efficiency.

The USB interface 230 is an interface that conforms to a USB standard specification, and may be specifically a mini USB interface, a micro USB interface, a USB Type-C interface, or the like. The USB interface 230 may be configured to connect to a charger to charge the electronic device 200, or may be configured to exchange data between the electronic device 200 and a peripheral device, or may be configured to connect to a headset for playing an audio through the headset. The interface may be further configured to connect to another electronic device like an AR device.

The charging management module 240 is configured to receive a charge input from the charger. The charger may be a wireless charger or a wired charger.

The power management module 241 is configured to connect to the battery 242, the charging management module 240, and the processor 210. The power management module 241 receives an input of the battery 242 and/or the charging management module 240, and supplies power to the processor 210, the internal memory 221, the display 294, the camera 293, the wireless communication module 260, and the like.

A wireless communication function of the electronic device 200 may be implemented by using the antenna 1, the antenna 2, the mobile communication module 250, the wireless communication module 260, a modem processor, a baseband processor, and the like.

The antenna 1 and the antenna 2 are configured to transmit and receive an electromagnetic wave signal. Each antenna in the electronic device 200 may be configured to cover one or more communication bands. Different antennas may be further multiplexed, to improve antenna utilization. For example, the antenna 1 may be multiplexed as a diversity antenna of a wireless local area network. In some other embodiments, the antenna may be used in combination with a tuning switch.

The mobile communication module 250 may provide a wireless communication solution that includes 2G/3G/4G/5G or the like and that is applied to the electronic device 200. The mobile communication module 250 may include at least one filter, a switch, a power amplifier, a low noise amplifier (low noise amplifier, LNA), and the like.

The wireless communication module 260 may provide a wireless communication solution that includes a wireless local area network (wireless local area network, WLAN) (for example, a wireless fidelity (wireless fidelity, Wi-Fi) network), Bluetooth (Bluetooth, BT), a global navigation satellite system (global navigation satellite system, GNSS), frequency modulation (frequency modulation, FM), a near field communication (near field communication, NFC) technology, an infrared (infrared, IR) technology, or the like and that is applied to the electronic device 200.

In some embodiments, in the electronic device 200, the antenna 1 and the mobile communication module 250 are coupled, and the antenna 2 and the wireless communication module 260 are coupled, so that the electronic device 200 can communicate with a network or another device by using a wireless communication technology.

The electronic device 200 implements a display function by using the GPU, the display 294, the application processor, and the like. The GPU is a microprocessor for image processing, and is connected to the display 294 and the application processor. The GPU is configured to: perform mathematical and geometric computation, and render an image. The processor 210 may include one or more GPUs that execute program instructions to generate or change display information.

The display 294 is configured to display an image, a video, and the like. The display 294 includes a display panel. In some embodiments, the electronic device 200 may include one or N displays 294, where N is a positive integer greater than 1. In some embodiments of this application, the display 294 may be configured to display a body temperature of the user.

The electronic device 200 may implement a photographing function by using the ISP, the camera 293, the video codec, the GPU, the display 294, the application processor, and the like.

The external memory interface 220 may be configured to be connected to an external storage card, for example, a micro SD card, to extend a storage capability of the electronic device 200. The external storage card communicates with the processor 210 through the external memory interface 220, to implement a data storage function. For example, files such as music and videos are stored in the external storage card.

The internal memory 221 may be configured to store computer-executable program code, where the executable program code includes instructions. The internal memory 221 may include a program storage area and a data storage area. In addition, the internal memory 221 may include a high-speed random access memory, and may further include a nonvolatile memory, for example, at least one magnetic disk storage device, a flash storage device, or a universal flash storage (universal flash storage, UFS). The processor 210 runs the instructions stored in the internal memory 221 and/or the instructions stored in the memory disposed in the processor, to perform various function applications and data processing of the electronic device 200.

The electronic device 200 may implement an audio function, like music playing and recording, by using the audio module 270, the application processor, and the like.

The audio module 270 is configured to convert digital audio information into an analog audio signal output, and is also configured to convert an analog audio input into a digital audio signal. The audio module 270 may be further configured to encode and decode an audio signal. The audio module 270 includes a loudspeaker, a receiver, a microphone, a headset jack, and the like. In some embodiments of this application, a loudspeaker in the audio module 270 may be configured to broadcast the body temperature of the user by voice.

The temperature sensor 280A is configured to detect a temperature. In some embodiments, the electronic device 200 executes a temperature processing policy based on the temperature detected by the temperature sensor 280A. For example, when a temperature reported by the temperature sensor 280A exceeds a threshold, the electronic device 200 lowers performance of a processor located near the temperature sensor 280A, to reduce power consumption and implement thermal protection. In some other embodiments, when the temperature is less than another threshold, the electronic device 200 heats the battery 242 to prevent the electronic device 200 from being shut down abnormally due to a low temperature. In some other embodiments, when the temperature is less than still another threshold, the electronic device 200 boosts an output voltage of the battery 242 to avoid abnormal shutdown due to a low temperature. In some embodiments of this application, the temperature sensor 280A may be installed in the electronic device 200, and is configured to detect a skin temperature of the user, or is configured to detect a temperature of a current environment. In some other embodiments of this application, a plurality of temperature sensors 280A may be installed in the electronic device 200. Some of the temperature sensors are configured to detect a skin temperature of the user, and some other temperature sensors 280A are configured to detect a temperature of a current environment and the like.

The gyroscope sensor 280B may be configured to determine a motion posture of the electronic device 200. In some embodiments, angular velocities of the electronic device 200 around three axes (namely, axes x, y, and z) may be determined by using the gyroscope sensor 280B. The gyroscope sensor 280B may be configured to implement image stabilization during photographing. For example, when a shutter is pressed, the gyroscope sensor 280B detects an angle at which the electronic device 200 jitters, calculates, based on the angle, a distance for which a lens module needs to compensate, and allows the lens to cancel the jitter of the electronic device 200 through reverse motion, to implement image stabilization. The gyroscope sensor 280B may be further used in a navigation scenario and a motion-sensing gaming scenario. In some embodiments of this application, the gyroscope sensor 280B may be configured to collect motion data of the user.

The acceleration sensor 280C may detect magnitudes of accelerations in various directions (generally on three axes) of the electronic device 200, and may detect a magnitude and a direction of gravity when the electronic device 200 is static. The acceleration sensor 280C may be further configured to identify a posture of the electronic device, and is used in an application like switching between a landscape mode and a portrait mode or a pedometer. In some embodiments of this application, the acceleration sensor 280C may also be configured to collect motion data of the user.

The PPG sensor 280D measures a heart rate and another biometric indicator by using a PPG technology. PPG is a method of shining light into skin and measuring light scattering generated due to blood flow. When blood flow dynamics change, for example, when a heart rate or a heart output changes, foreseeable scattering occurs in light entering a human body. In some embodiments of this application, the processor 210 may determine a heart rate of the user by processing a signal sensed by the PPG sensor.

The touch sensor 280E is also referred to as a "touch control component". The touch sensor 280E may be disposed on the display 294. The touch sensor 280E and the display 294 form a touchscreen, and the touchscreen is also referred to as a "touch control screen". The touch sensor 280E is configured to detect a touch operation performed on or near the touch sensor 280E. The touch sensor may transfer the detected touch operation to the application processor, to determine a type of a touch event. A visual output related to the touch operation may be provided through the display 294. In some other embodiments, the touch sensor 280E may alternatively be disposed on a surface of the electronic device 200, and is located on a position different from that of the display 294.

Optionally, the sensor module 280 may further include a pressure sensor, a barometric pressure sensor, a magnetic sensor, a distance sensor, an optical proximity sensor, a fingerprint sensor, an ambient light sensor, a bone conduction sensor, and the like.

The button 290 includes a power button, a volume button, and the like. The button 290 may be a mechanical button, or may be a touch button. The electronic device 200 may receive a button input, and generate a button signal input related to user settings and function control of the electronic device 200.

The motor 291 may generate a vibration prompt. The motor 291 may be configured to provide an incoming call vibration prompt or a touch vibration feedback.

The indicator 292 may be an indicator light, and may be configured to indicate a charging status and a power change, or may be configured to indicate a message, a missed call, a notification, and the like.

The SIM card interface 295 is configured to connect to a SIM card. The SIM card may be inserted into the SIM card interface 295 or removed from the SIM card interface 295, to implement contact with or separation from the electronic device 200.

A software system of the electronic device 200 may use a layered architecture, an event-driven architecture, a microkernel architecture, a micro service architecture, or a cloud architecture. In this embodiment of the present invention, an Android system with the layered architecture is used as an example to illustrate a software structure of the electronic device 200.

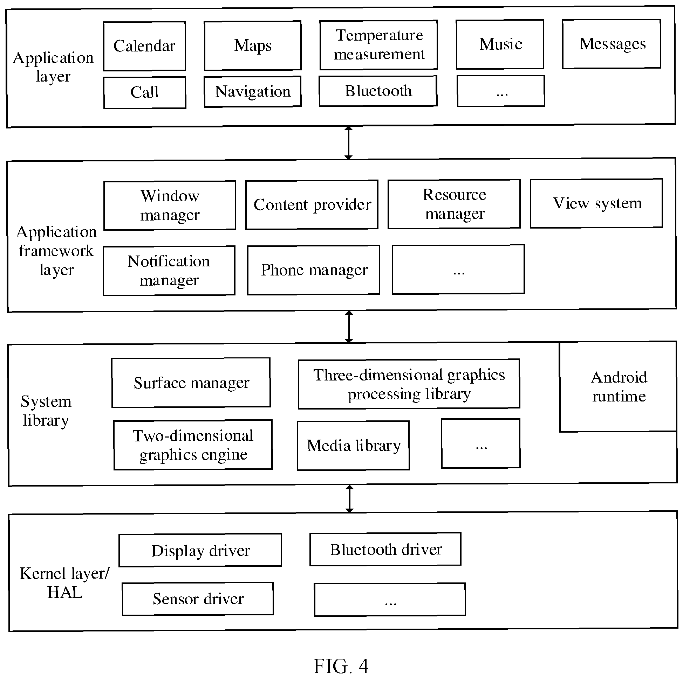

FIG. 4 is a block diagram of a software structure of an electronic device 200 according to an embodiment of this application.

In a layered architecture, software is divided into several layers, and each layer has a clear role and a clear task. The layers communicate with each other through a software interface. In some embodiments, an Android system is divided into four layers: an application layer, an application framework layer, an Android runtime (Android runtime) and system library, and a kernel layer from top to bottom.

The application layer may include a series of application packages.

As shown in FIG. 4, the application packages may include applications such as Calendar, Phone, Maps, Navigation, Bluetooth, Music, Messages, and Temperature measurement.

The application framework layer provides an application programming interface (application programming interface, API) and a programming framework for an application at the application layer. The application framework layer includes some predefined functions.

As shown in FIG. 4, the application framework layer may include a window manager, a content provider, a view system, a phone manager, a resource manager, a notification manager, and the like.

The window manager is configured to manage a window program. The window manager may obtain a size of a display, perform screen locking, take a screenshot, and the like.

The content provider is configured to: store and obtain data, and enable the data to be accessed by an application. The data may include a video, an image, an audio, calls that are made and answered, a browsing history and a browsing bookmark, an address book, and the like.

The view system includes visual controls such as a control for displaying a text and a control for displaying an image. The view system may be configured to construct an application. A display interface may include one or more views. For example, a display interface including a message notification icon may include a text display view and an image display view.

The phone manager is configured to provide a communication function of the electronic device 200, for example, management of a call status (including answering, declining, or the like).

The resource manager provides various resources such as a localized character string, an icon, an image, a layout file, and a video file for an application.

The notification manager enables an application to display notification information in a status bar, and may be configured to convey a notification message. The displayed notification information may automatically disappear after a short pause without user interaction. For example, the notification manager is configured to: notify download completion, provide a message notification, and the like. The notification manager may alternatively be a notification that appears in a top status bar of the system in a form of a graph or a scroll bar text, for example, a notification of an application that is run in the background, or may be a notification that appears on a screen in a form of a dialog window. For example, text information is displayed in the status bar, an announcement is given, the electronic device vibrates, or an indicator light blinks.

The Android runtime includes a kernel library and a virtual machine. The Android runtime is responsible for scheduling and management of the Android system.

The kernel library includes two parts: a function that needs to be invoked in Java language and a kernel library of Android.

The application layer and the application framework layer run on the virtual machine. The virtual machine executes java files at the application layer and the application framework layer as binary files. The virtual machine is used to perform functions such as object lifecycle management, stack management, thread management, security and exception management, and garbage collection.

The system library may include a plurality of functional modules, for example, a surface manager (surface manager), a media library (Media Library), a three-dimensional graphics processing library (for example, OpenGL ES), and a 2D graphics engine (for example, SGL).

The surface manager is configured to manage a display subsystem, and provides fusion of 2D and 3D layers for a plurality of applications.

The media library supports playback and recording in a plurality of commonly used audio and video formats, static image files, and the like. The media library may support a plurality of audio and video encoding formats, for example, MPEG-4, H.264, MP3, AAC, AMR, JPG, and PNG.

The three-dimensional graphics processing library is configured to implement three-dimensional graphics drawing, image rendering, composition, layer processing, and the like.

The 2D graphics engine is a drawing engine for 2D drawing.

The kernel layer is a layer between hardware and software. The kernel layer includes at least a display driver, a sensor driver, and a Bluetooth driver.

The following describes an example of a working process of software and hardware of the electronic device 200 with reference to a body temperature measurement scenario. When the touch sensor 280E receives a touch operation, a corresponding hardware interruption is sent to the kernel layer. The kernel layer processes the touch operation into an original input event (including touch coordinates and timestamp information of the touch operation). The original input event is stored at the kernel layer. The application framework layer obtains the original input event from the kernel layer, and identifies a control corresponding to the input event. For example, the touch operation is a tap operation, and a control corresponding to the tap operation is a control for enabling a temperature measurement function. A temperature measurement application invokes an interface of the application framework layer, obtains the original input event, enables the temperature measurement function, invokes the kernel layer to enable the sensor driver, and drives a sensor (for example, an acceleration sensor, a gyroscope sensor, or a PPG sensor) to obtain a target parameter, where the target parameter includes motion data of a user, a current heart rate value of the user, and the like. The motion data of the user is used to determine a current motion status of the user. The processor 210 determines a body temperature of the user based on the current motion status of the movement and the current heart rate value of the user. Then, the display driver at the kernel layer is enabled, to drive the display 294 to display the body temperature of the user; and/or an audio driver at the kernel layer is enabled, to drive a loudspeaker in the audio module 270 to broadcast the body temperature of the user by voice.

In some embodiments, when a mapping relationship between different motion statuses and heart rate baselines of the user is obtained, the processor 210 determines a corresponding heart rate baseline based on the current motion status of the user and the mapping relationship that are obtained by the sensor, and calculates the body temperature of the user based on the current heart rate value of the user and the determined heart rate baseline. Then, the display driver at the kernel layer is enabled, to drive the display 294 to display the body temperature of the user; and/or an audio driver at the kernel layer is enabled, to drive a loudspeaker in the audio module 270 to broadcast the body temperature of the user by voice.

The following first describes, by using an example in which the electronic device is a smart band, the body temperature measurement method provided in embodiments of this application with reference to the accompanying drawings.

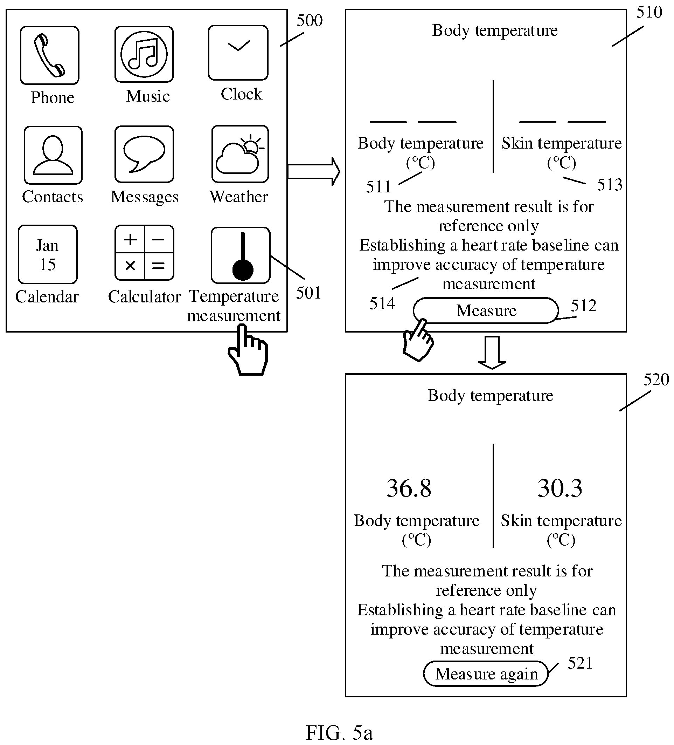

A plurality of applications are installed in the smart band, and different applications may be used to implement different functions. For example, an interface 500 shown in FIG. 5a includes a temperature measurement application 501, and the temperature measurement application 501 may implement a body temperature measurement function. It should be noted that names of the applications mentioned in this application do not constitute a limitation on functions of the applications, and the applications may have other names. This is not limited in this application, and a uniform description is provided herein.

The smart band receives an operation performed by the user to open the temperature measurement application 501 by the user, for example, a tapping operation performed by the user on an icon of the temperature measurement application 501. In response to the operation, the smart band may present a body temperature measurement interface 510. The body temperature measurement interface 510 includes a body temperature display area 511 and a body temperature measurement button 512. The body temperature display area may be used to display a current body temperature, a historical body temperature, and the like of the user. The body temperature measurement button 512 may be configured to enable a body temperature measurement function.

Optionally, the body temperature measurement interface 510 may further include another display area, for example, a skin temperature display area 513, a warm reminder display area 514, or a heart rate display area. This is not limited in this application. The skin temperature display area 513 may be configured to display a temperature (for example, a skin temperature of a part on which the smart band is worn) of partial skin of the user, the warm reminder display area 514 may be configured to display a warm reminder for the user, and the heart rate display area may be configured to display a current heart rate of the user and the like.

The smart band detects an operation performed by the user to enable the body temperature measurement function, for example, a tapping operation on the body temperature measurement button 512. In response to the operation of the user, the smart band obtains a target parameter, where the target parameter includes a current motion status of the user, a current heart rate value of the user, and the like. In some embodiments, the smart band can obtain a mapping relationship between different motion statuses and heart rate baselines of the user. In this case, the smart band may determine, based on an obtained current motion status of the user and the mapping relationship, a heart rate baseline corresponding to the current motion status of the user, where the determined heart rate baseline indicates a heart rate status of the user in the motion status. Then, the smart band calculates a body temperature of the user based on the obtained current heart rate value of the user and the determined heart rate baseline, and displays the body temperature of the user. For example, the smart band may present an interface 520 to display the body temperature of the user. As shown on the interface 520, the measured body temperature is 36.8°C. Optionally, when the target parameter includes a skin temperature of a target part of the user, the skin temperature may be further displayed. As shown on the interface 520, the measured skin temperature is 30.3°C. Optionally, after the user uses the temperature measurement function, the temperature measurement button 512 may be further presented in a form of a button 521.

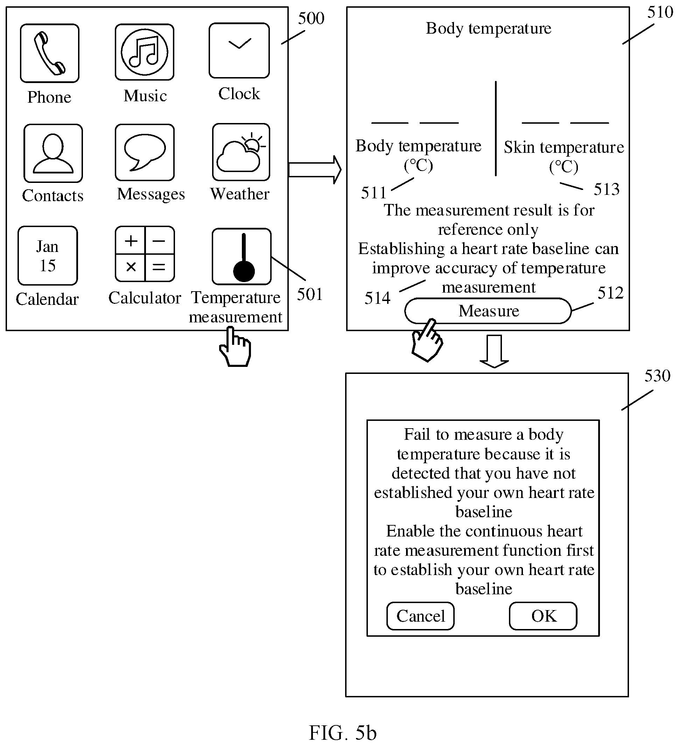

In some other embodiments, the smart band does not obtain the mapping relationship between different motion statuses and heart rate baselines of the user. In this case, the smart band does not enable the body temperature measurement function, and does not display the measured body temperature of the user. Optionally, to help the user learn of this situation, the smart band may display a first reminder message, to remind the user that the body temperature measurement cannot be performed because the mapping relationship between motion statuses and heart rate baselines is not established currently. Optionally, the smart band may further display a second reminder message, to remind the user to establish the mapping relationship between motion statuses and heart rate baselines of the user. The second reminder message and the first reminder message may be presented simultaneously, or may be presented successively. The second reminder message and the first reminder message may be presented on a same display interface, or may be presented on different display interfaces. The second reminder message may be presented in a manner of an entire display window, or may be presented in a manner of a pop-up prompt window. For example, as shown in FIG. 5b, the second reminder message may be shown as a message on an interface 530. A manner of establishing a heart rate baseline is not described herein, and is described in detail in the following.

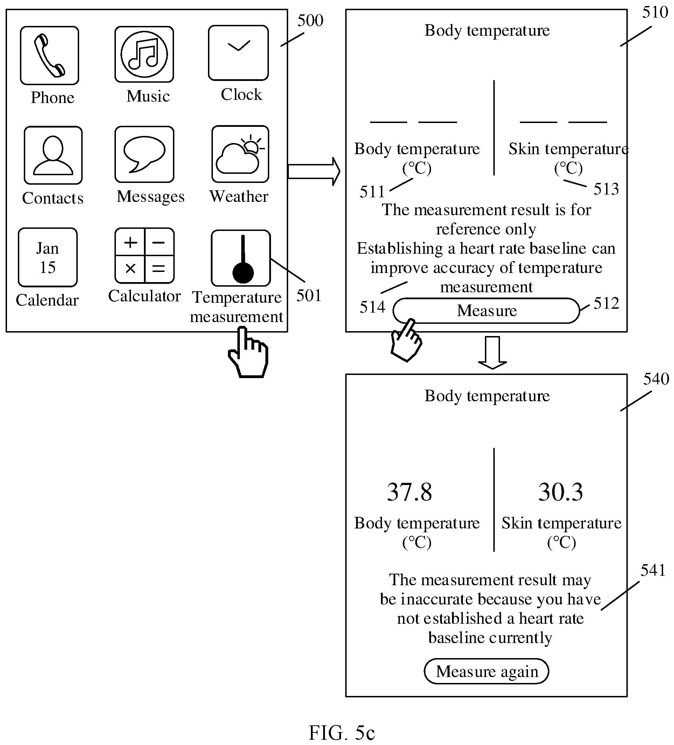

In still some other embodiments, the smart band does not obtain the mapping relationship between different motion statuses and heart rate baselines of the user. In this case, the smart band may directly calculate a body temperature of the user based on the obtained current motion status of the user and the current heart rate value of the user, and display the body temperature of the user. For example, as shown in FIG. 5c, in this case, the smart band may present an interface 540 to display the body temperature of the user. It should be noted that, compared with that in the implementation in FIG. 5a, the body temperature that is of the user and that is obtained through measurement in this implementation may be inaccurate. Therefore, the body temperature displayed on the interface 540 may be different from the body temperature displayed on the interface 520. Optionally, to help the user learn that the measured body temperature in this case may be inaccurate, the smart band may further display a third reminder message, to remind the user that the measured body temperature may be inaccurate because the mapping relationship between motion statuses and heart rate baselines is not established currently. For example, the smart band may remind the user by using a prompt message 541 shown on the interface 540.

Optionally, in this embodiment, the smart band may also display the second reminder message mentioned in the implementation in FIG. 5b, to remind the user to establish the mapping relationship between motion statuses and heart rate baselines of the user. A display location (for example, displayed on the interface 510 or the interface 540), a display manner (for example, presented in a manner of an entire interface or presented in a manner of a prompt message), a display occasion (for example, displayed before or after the display interface 540), and the like of the second reminder message are not limited in this application.

It should be noted that the smart band may directly display the first reminder message, the second reminder message, and the third reminder message, or may send the first reminder message, the second reminder message, and the third reminder message to another electronic device, for example, a mobile phone, and display the first reminder message, the second reminder message, and the third reminder message on the mobile phone, to remind the user. Alternatively, both the smart band and the mobile phone display the reminder messages.

Optionally, there may be a binding relationship between the smart band and the another electronic device. A binding method includes but is not limited to a Bluetooth technology, a Wi-Fi network, a tap, a distributed soft bus, a near field communication (near field communication, NFC) technology, a ZigBee (ZigBee), and the like. Alternatively, a communication connection relationship may be established between the smart band and the another electronic device, so that the smart band can send instructions to the another electronic device. This is not limited in this application.

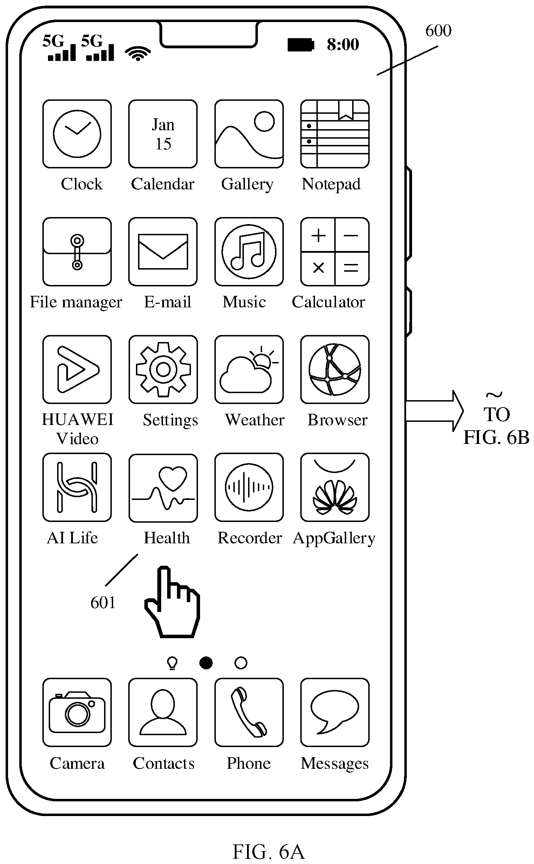

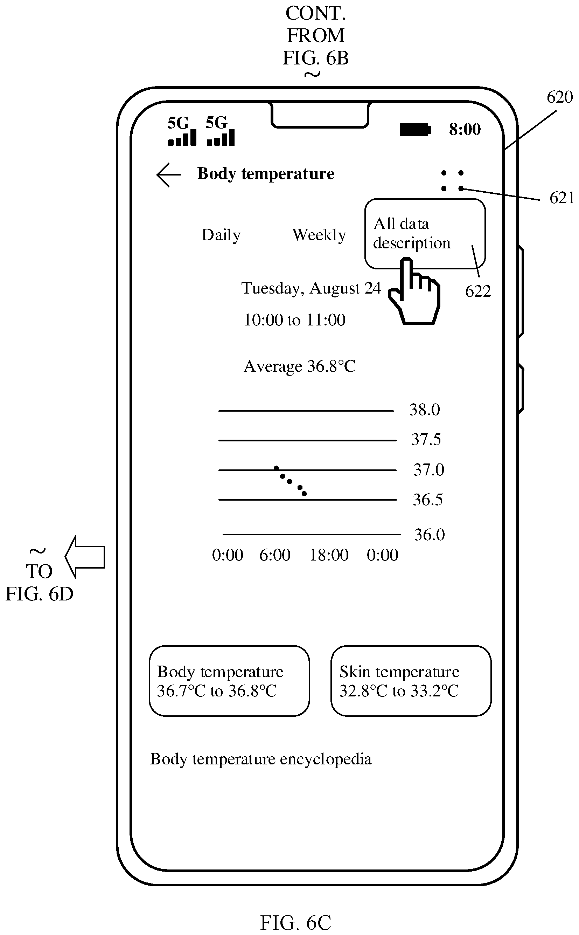

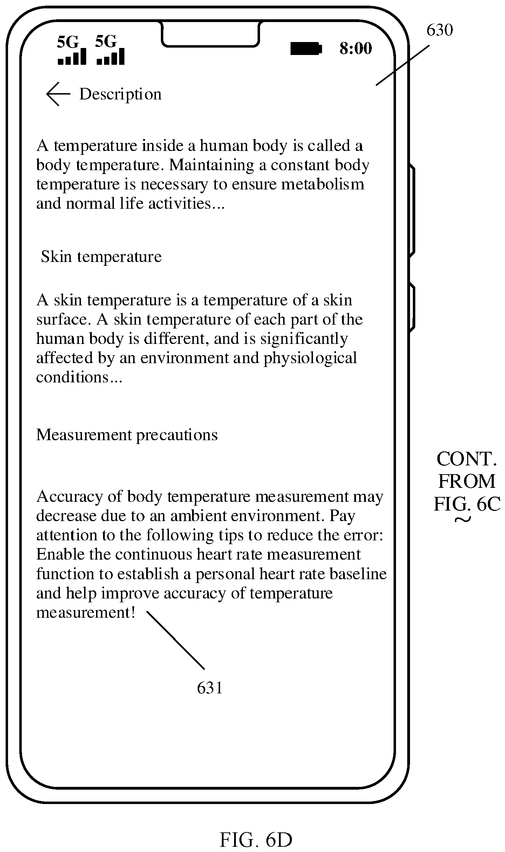

For example, the another electronic device is a mobile phone. FIG. 6A to FIG. 6D is a schematic diagram of a mobile phone interface on which the reminder messages are displayed according to an embodiment of this application.

It is assumed that the mobile phone is successfully bound to the smart band, for example, bound by using a health application in the mobile phone. Because a method for binding the smart band to the mobile phone by using the health application in the mobile phone belongs to the conventional technology, details are not described herein again. The mobile phone receives an operation of opening the health application by the user, for example, a tap operation performed by the user on a health application icon 601 on an interface 600. In response to the operation, the mobile phone may present an interface 610, and the interface 610 includes a plurality of cards such as a heart health card, a sleep card, and a body temperature card. Different cards may be used to view different data and/or different functions. When detecting a tapping operation performed by the user on a body temperature card 611, the mobile phone may present an interface 620. The interface 620 includes body temperature data of the user, and the body temperature data may be synchronized by the mobile phone from the smart band. In some possible implementations, the second reminder message may be displayed on the interface 620 (not shown in the figure). In some other possible implementations, the second reminder message may alternatively be displayed on another interface. For example, when detecting a tap operation performed by the user on a description option 622 in a function button 621, the mobile phone may present an interface 630. The second reminder message may be displayed on the interface 630, as shown in a reminder message 631. A display location, display time, a display manner, and the like of the second reminder message are not limited in this application.

It should be noted that the foregoing implementations may be separately used, or may be used in combination.

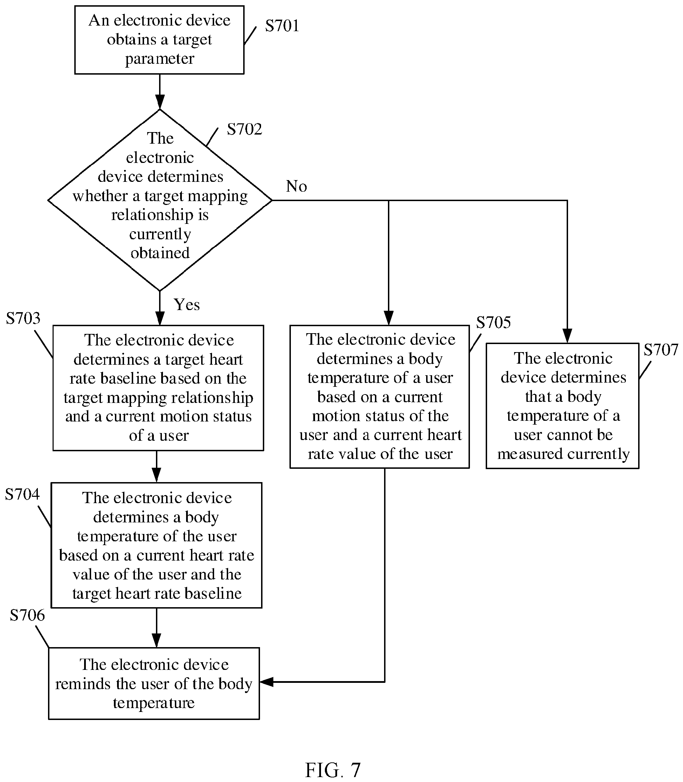

For example, FIG. 7 shows a body temperature measurement method according to an embodiment of this application. The method includes the following steps.

S701: An electronic device obtains a target parameter.

In a possible implementation, an operation of obtaining the target parameter by the electronic device may be performed in real time or periodically. For example, the electronic device detects one operation of enabling a body temperature measurement function by a user. In response to the operation, the electronic device may obtain the target parameter in real time or periodically within a predetermined time period.

In another possible implementation, the operation of obtaining the target parameter by the electronic device needs to be triggered by the user each time. For example, the electronic device detects one operation of enabling the body temperature measurement function by the user. In response to the operation, the electronic device performs one operation of obtaining the target parameter.

It may be understood that the electronic device may measure the target parameter, or another electronic device may measure the target parameter, and then the electronic device obtains the target parameter from the another electronic device. This is not specifically limited in this application.

Optionally, the operation of enabling the body temperature measurement function includes but is not limited to an operation (like tapping, double tapping, touching and holding, or sliding) on a physical button or a virtual button, a voice operation, a gesture operation, or the like. For example, the operation of enabling the body temperature measurement function of the electronic device may be a tapping operation performed on the body temperature measurement button 512 shown in FIG. 5a.

The target parameter includes motion data of the user, a current heart rate value of the user, and the like. The motion data of the user is used to determine a current motion status of the user.

In a possible implementation, motion statuses may include a sleep state and a non-sleep state.

Optionally, the non-sleep state may further include a low activity amount state, a medium activity amount state, a high activity amount state, and the like. A level of an activity amount reflects a motion intensity of the user. The low activity amount state is a state in which an activity amount of the user is less than a first threshold, the medium activity amount state is a state in which an activity amount of the user is greater than or equal to the first threshold and less than or equal to a second threshold, and the high activity amount state is a state in which an activity amount of the user is greater than the second threshold. The first threshold and the second threshold may be set based on an actual requirement.

In a possible implementation, the activity amount of the user may be determined based on collected motion data of the user. For example, the electronic device collects motion signals of the user by using an acceleration sensor, a gyroscope sensor, or the like, generates the motion data of the user, and determines the activity amount of the user based on the motion data.

For example, statistics are collected on motion data generated in a specific time period. When a statistical value is less than the first threshold, it may be determined that the user is in the low activity amount state; when a statistical value is greater than or equal to the first threshold and less than or equal to the second threshold, it may be determined that the user is in the medium activity amount state; or when a statistical value is greater than the second threshold, it may be determined that the user is in the high activity amount state. The statistical value may be various types of statistical data such as an average value, a mode, a median, and a total value of the motion data.

In another possible implementation, the activity amount of the user may be further determined based on various factors such as a motion type (for example, running, walking, riding, or rope skipping), motion duration, a motion intensity, and a motion distance of the user. For example, if the user is walking slowly, and the user has currently moved for 40 minutes, and/or the user has currently walked for 2 kilometers, it may be determined that the user is in the low activity amount state. If the user is running fast, and the user has currently moved for 3 minutes, and/or the user has currently run for 0.5 kilometers, it may be determined that the user is in a high activity amount state. If the user is skipping a rope, and when the user has currently moved for 15 minutes, and/or a quantity of rope skipping times of the user has been 200, it may be determined that the user is in the high activity amount state, or the like.

It should be noted that the motion statuses may be further classified in another manner. For example, the motion statuses include a slow-walking state, a fast-walking state, a jogging state, a fast-running state, a riding state, a rope skipping state, and the like. A specific classification manner of the motion statuses is not limited in this application.

Optionally, the target parameter further includes one or more of a skin temperature of a target part of the user and a current ambient temperature. For example, the skin temperature of the target part of the user may be a skin temperature of a part on which the user wears the electronic device, or may be a skin temperature of another part of the user. It should be understood that the skin temperature can reflect only a temperature of a part of the user, and may not accurately reflect a real body temperature of the user.

S702: The electronic device determines whether a target mapping relationship is currently obtained.

A mapping relationship is a mapping relationship between different motion statuses and heart rate baselines. The target mapping relationship is a mapping relationship between different motion statuses and heart rate baselines of the user. Optionally, the motion statuses and the heart rate baselines may be in a one-to-one relationship, or may be in a one-to-many relationship (for example, one heart rate baseline corresponds to a plurality of motion statuses). This is not limited in this application.

In some embodiments, the electronic device may establish and store the target mapping relationship, and then obtain the target mapping relationship locally or from a server. In some other embodiments, the electronic device may alternatively obtain the target mapping relationship from another electronic device. A manner in which the electronic device obtains the target mapping relationship is not specifically limited in this application.

It should be noted that an execution sequence of step S701 in which the target parameter is obtained and step S702 is not limited in this application.

If the target mapping relationship is obtained, steps S703, S704, and S706 are performed. If no target mapping relationship is obtained, steps S705 and S706 are performed, or step S707 is performed.

S703: The electronic device determines a target heart rate baseline based on the target mapping relationship and the current motion status of the user.

The target heart rate baseline indicates a heart rate status of the user in the motion status.

Optionally, before step S703, the method shown in FIG. 7 further includes steps S703a and S703b (not shown in the figure).

S703a: Obtain account information of the user.

The account information may be account information for logging in to a target application, and the target application may be used to implement a function of measuring a body temperature of the user. For example, the target application may be the temperature measurement application 501 shown in FIG. 5a, the health application 601 shown in FIG. 6A, or the like. Current login account information may be stored by the electronic device, for example, account information for logging in to the temperature measurement application 501 shown in FIG. 5a; or may be obtained by the electronic device from another electronic device, for example, account information for logging in to the health application 601 shown in FIG. 6A. It should be understood that before the user uses a function of each of the temperature measurement application shown in FIG. 5a and the health application shown in FIG. 6A, the user needs to first log in to a corresponding application by using the account information of the user.

S703b: Obtain, based on the account information, the target mapping relationship corresponding to the user.

For example, when the mapping relationship is established, the mapping relationship is associated with the account information for logging in to the target application. The electronic device may determine, based on the current account information for logging in to the target application, whether the account has the target mapping relationship, that is, whether a mapping relationship corresponding to the user exists.

S704: The electronic device determines a body temperature of the user based on the current heart rate value of the user and the target heart rate baseline.

Optionally, a first personalized body temperature algorithm model is preset in the electronic device. In this case, the electronic device may input the current heart rate value and the target heart rate baseline of the user into the first personalized body temperature algorithm model, and the first personalized body temperature algorithm model outputs a body temperature value of the user after processing the current heart rate value and the target heart rate baseline of the user. The first personalized body temperature algorithm model may be obtained through model training. A specific algorithm used by the model is not limited in this application. For a specific model training method, refer to the conventional technology.

Optionally, the first personalized body temperature algorithm model may be alternatively preset in another electronic device. The electronic device sends the current motion status of the user, the current heart rate value and/or the target heart rate baseline of the user, and the like to the another electronic device, and the another electronic device calculates the body temperature of the user. Then, the electronic device receives the body temperature of the user from the another electronic device.

Optionally, when the target parameter further includes the skin temperature of the target part of the user and/or the current ambient temperature, the body temperature of the user may be further determined based on the current heart rate value and the target heart rate baseline of the user, the skin temperature of the target part of the user, and/or the current ambient temperature. Similar to a case in which the body temperature of the user is determined based on the current heart rate value and the target heart rate baseline of the user, the current heart rate value and the target heart rate baseline of the user, the skin temperature of the target part of the user, and/or the current ambient temperature may alternatively be input into a preset second personalized body temperature algorithm model. The second personalized body temperature algorithm model outputs a body temperature value of the user after processing the current heart rate value and the target heart rate baseline of the user, the skin temperature of the target part of the user, and/or the current ambient temperature. A preset location of the second personalized body temperature algorithm model is also not limited in this application.

S705: The electronic device determines a body temperature of the user based on the current motion status of the user and the current heart rate value of the user.

Optionally, a first general body temperature algorithm model may be further preset inside the electronic device. The electronic device may input the current motion status of the user and the current heart rate value of the user into the first general body temperature algorithm model, and the first general body temperature algorithm model outputs a body temperature of the user after processing the current motion status of the user and the current heart rate value of the user. The first general body temperature algorithm model may also be obtained through model training. The first general body temperature algorithm model is obtained by collecting statistics on heart rate characteristics of most users.

Optionally, the first general body temperature algorithm model may be alternatively preset in another electronic device. The electronic device sends the current motion status of the user and the current heart rate value of the user to the another electronic device, and the another electronic device calculates the body temperature of the user. Then, the electronic device receives the body temperature of the user from the another electronic device.

Optionally, when the target parameter further includes the skin temperature of the target part of the user and/or the current ambient temperature, the body temperature of the user may be further determined based on the current motion status of the user, the current heart rate value of the user, the skin temperature of the target part of the user, and/or the current ambient temperature. Similar to a case in which the body temperature of the user is determined based on the current motion status of the user and the current heart rate value of the user, the current motion status of the user, the current heart rate value of the user, the skin temperature of the target part of the user, and/or the current ambient temperature may alternatively be input into a preset second general body temperature algorithm model. The second general body temperature algorithm model outputs a body temperature value of the user after processing the current motion status of the user, the current heart rate value of the user, the skin temperature of the target part of the user, and/or the current ambient temperature. A preset location of the second general body temperature algorithm model is also not limited in this application.

S706: The electronic device reminds the user of the body temperature.

For example, the electronic device may display the interface 520 shown in FIG. 5a or the interface 540 shown in FIG. 5c, to display the body temperature of the user; and/or the electronic device may broadcast the body temperature of the user by voice. A manner used by the electronic device to remind the user of the body temperature is not limited in this application.

Optionally, the electronic device may display the body temperature of the user, or the electronic device may send the body temperature of the user to another electronic device, and the another electronic device displays the body temperature of the user.

Optionally, the electronic device may further display a second reminder message, to remind the user to establish the mapping relationship between motion statuses and heart rate baselines of the user. For example, the second reminder message may be shown as a warm reminder "Establishing a heart rate baseline may improve temperature measurement accuracy" on the interface 510 shown in FIG. 5a, "Please first enable a continuous heart rate measurement function to establish your own heart rate baseline" on the interface 530 shown in FIG. 5b, or the like.

S707: The electronic device determines that the body temperature of the user cannot be measured currently.

For example, the electronic device may present the interface 530 shown in FIG. 5b, to remind the user that the body temperature cannot be measured; and/or the electronic device may remind, in a voice broadcast manner, the user that the body temperature cannot be measured currently. Optionally, the electronic device may further display a second reminder message, to remind the user to establish the mapping relationship between motion statuses and heart rate baselines of the user.

Based on the foregoing technical solution, the body temperature of the user is estimated based on a plurality of physiological parameters such as the current motion status of the user, the current heart rate, and the heart rate baseline corresponding to the motion status, so that the estimated body temperature is more accurate, and user experience is improved. In addition, each user may have a different heart rate characteristic, that is, may have a different heart rate baseline. In addition, a same user may also have different heart rate baselines in different motion statuses. Therefore, the estimated body temperature is enabled to be more accurate in a manner of establishing a heart rate baseline.

With reference to the accompanying drawings, the following describes a process of establishing the mapping relationship (namely, the target mapping relationship) between the different motion statuses and the heart rate baselines of the user.

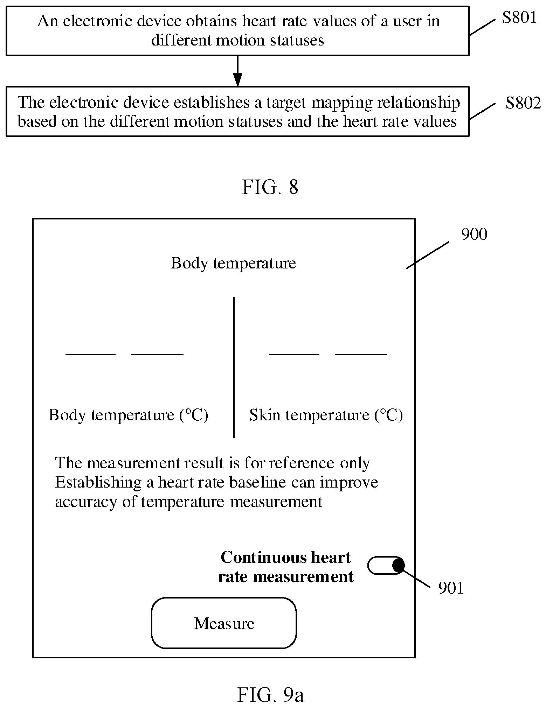

For example, FIG. 8 shows a method for establishing a mapping relationship between different motion statuses and heart rate baselines of a user according to an embodiment of this application. The method includes the following steps.

S801: An electronic device obtains heart rate values of a user in different motion statuses.

Optionally, before the heart rate values of the user in the different motion statuses are obtained, a continuous heart rate monitoring function of the electronic device needs to be first enabled. The continuous heart rate monitoring function may be used to continuously measure a heart rate of the user. Optionally, the continuous heart rate monitoring function is enabled by default, or may be triggered by the user to be enabled.

In some embodiments, an operation of obtaining the heart rate values of the user in the different motion statuses may be periodically performed. For example, the electronic device detects one operation of enabling the continuous heart rate monitoring function by the user. In response to the operation, the electronic device may periodically (for example, every one month or every three months) perform, within a predetermined time period, the operation of obtaining the heart rate values of the user in the different motion statuses.

In some other embodiments, the operation of obtaining the heart rate values of the user in the different motion statuses needs to be triggered by the user each time. For example, the electronic device detects one operation of enabling the continuous heart rate monitoring function by the user. In response to the operation, the electronic device performs one operation of obtaining the heart rate values of the user in the different motion statuses.

In a possible implementation, the user may directly operate (for example, operate a button that is on the electronic device and that is used to enable the continuous heart rate monitoring function) the electronic device, to enable the continuous heart rate monitoring function.

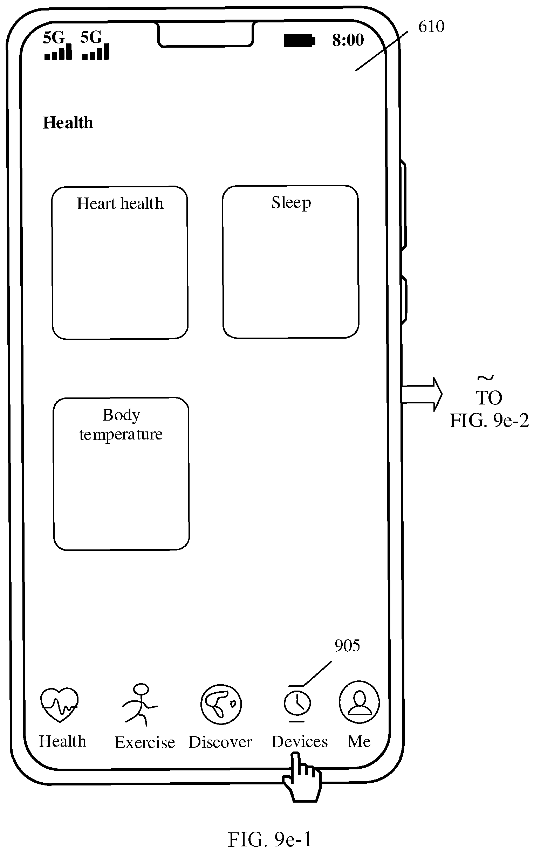

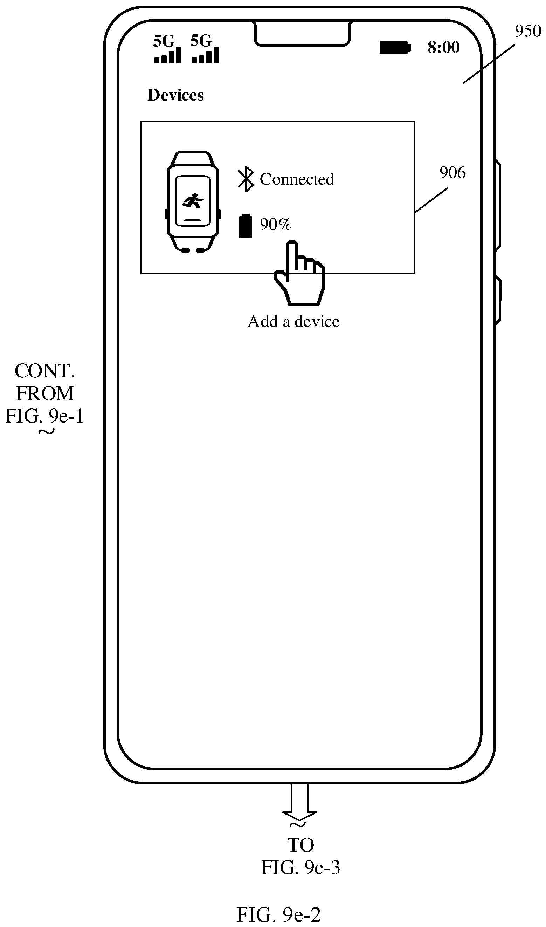

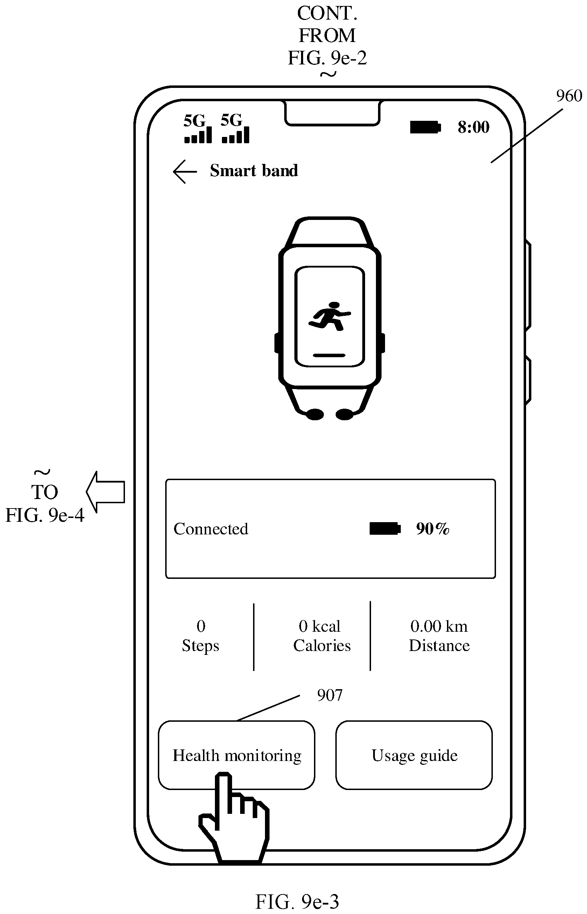

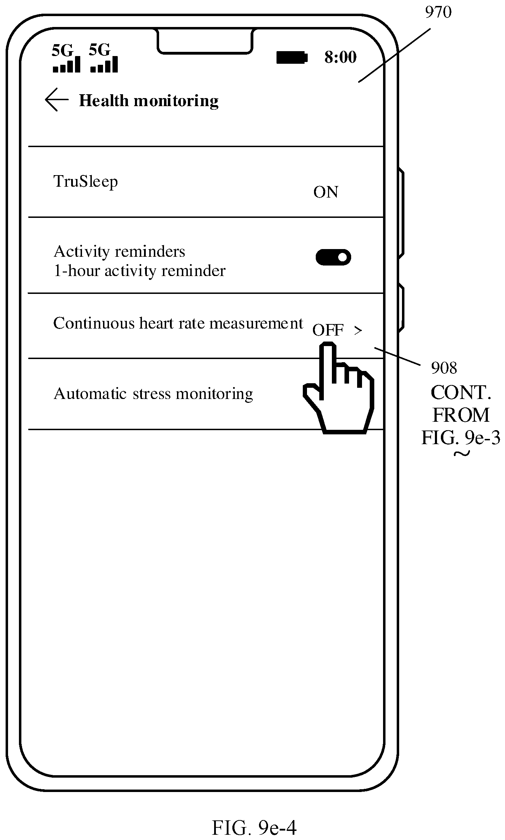

For example, as shown in FIG. 9a, a temperature measurement interface 900 of the electronic device includes a function button 901 for continuously measuring a heart rate. When an enabling operation performed by the user on the button 901 is detected, the continuous heart rate monitoring function of the electronic device is enabled.

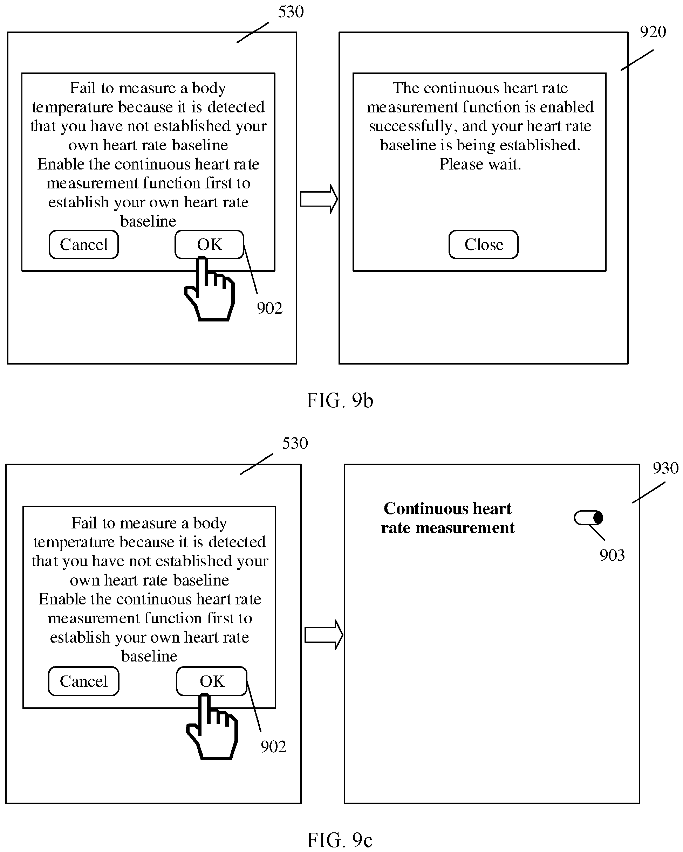

For another example, as shown in FIG. 9b, with reference to the implementation in FIG. 5b, the electronic device cannot perform body temperature measurement when the user does not establish a heart rate baseline of the user. The electronic device may display a second reminder message, to remind the user to establish the heart rate baseline of the user. The second reminder message includes a prompt that the continuous heart rate monitoring function needs to be enabled to establish the heart rate baseline of the user. The user may directly enable the continuous heart rate monitoring function of the electronic device based on the second reminder message. For example, based on the interface 530, when a tapping operation performed by the user on an OK button 902 is detected, the electronic device may directly enable the heart rate monitoring function. Optionally, the electronic device may further remind the user that the continuous heart rate monitoring function is enabled, that a heart rate baseline is being established, or the like. For example, the electronic device may present an interface 920 to prompt the user. Optionally, the electronic device may further remind the user of an approximate time period required for establishing the heart rate baseline, so that the user learns of a specific situation, and user experience is improved.

For another example, as shown in FIG. 9c, when a tapping operation performed by the electronic device on the OK button 902 is detected, the electronic device jumps to an interface 930 used to enable the continuous heart rate monitoring function. The interface 930 includes a function button 903 for continuously monitoring a heart rate. When an enabling operation performed by the user on the function button 903 is detected, the electronic device enables the continuous heart rate monitoring function.

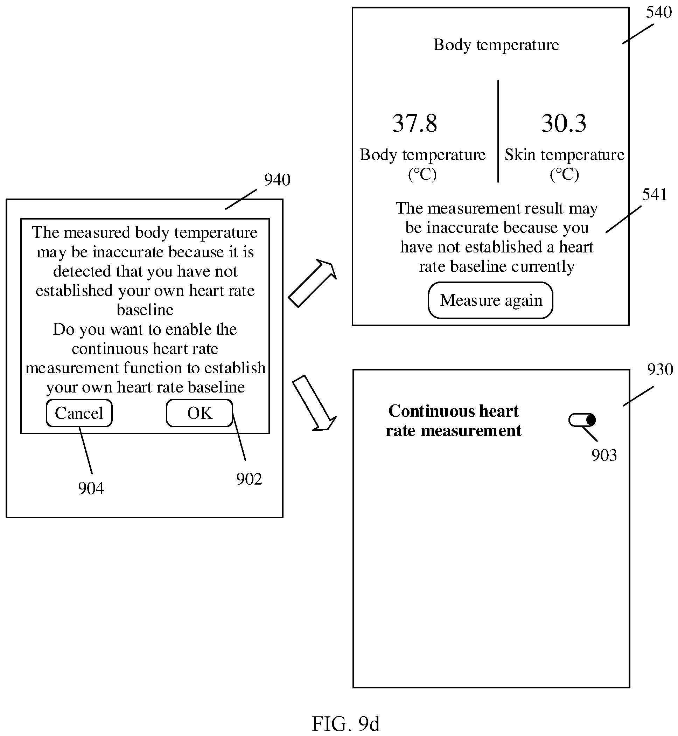

For another example, as shown in FIG. 9d, with reference to the implementation in FIG. 5c, the electronic device may also perform body temperature measurement when the user does not establish a heart rate baseline of the user. However, in this case, accuracy of a body temperature obtained through measurement is low. Before the electronic device presents the interface 540 shown in FIG. 5c, the electronic device may first display the second reminder message, to remind the user that the user may first establish the heart rate baseline of the user and then measure the body temperature, so as to improve accuracy of the body temperature obtained through measurement. For example, the second reminder message displayed by the electronic device may be shown on the interface 940. When a tap operation performed by the user on a cancel button 904 is detected, the electronic device may present a body temperature display interface 540. When a tap operation performed by the user on the OK button 902 is detected, the electronic device may directly enable the heart rate monitoring function based on the implementation in FIG. 9b. Alternatively, the electronic device may use the manner described in FIG. 9c. The electronic device first jumps to the interface 930 used to enable the heart rate monitoring function, and then enables the heart rate monitoring function in response to an enabling operation performed on the function button 903 for continuously monitoring a heart rate.

In another possible implementation, the user may operate another electronic device, so that the another electronic device controls enabling of the continuous heart rate monitoring function of the electronic device.

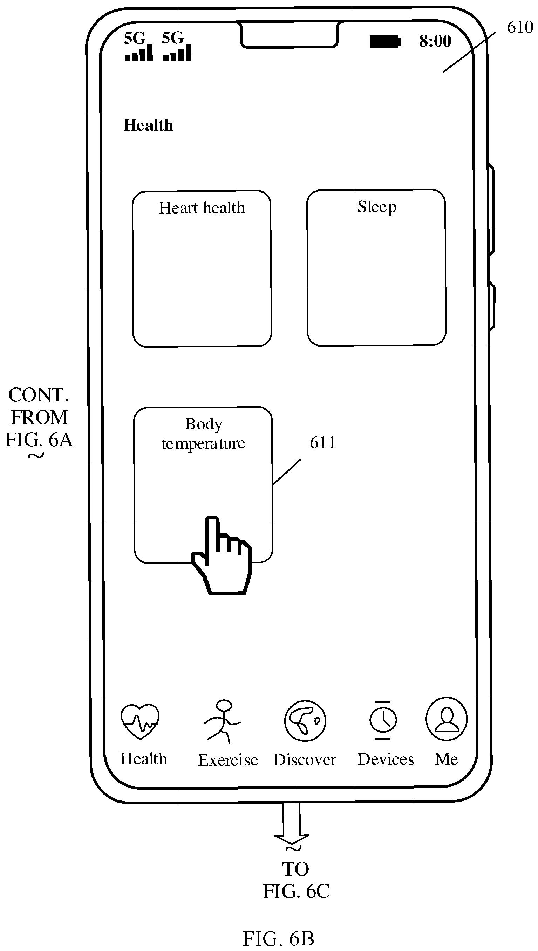

In this implementation, for example, as shown in FIG. 9e-1 to FIG. 9e-4, an example in which the another electronic device is a mobile phone, the electronic device is a smart band, and the mobile phone is successfully bound to the smart band is still used. Based on the interface 610 shown in FIG. 6B, when a tap operation performed by the user on a device button 905 is detected, the mobile phone may present an interface 950. An electronic device that is currently successfully bound may be displayed on the interface 950. When a tapping operation performed by the user on a smart band 906 is detected, the mobile phone may present an interface 960. The interface 960 may be configured to display usage data of the smart band and the like. The user may manage the smart band by using a health monitoring button 907. In response to a tapping operation performed by the user on the health monitoring button 907, the mobile phone may present an interface 970. The interface 970 may be used to enable or disable some functions of the smart band. For example, when a tap operation performed by the user on a continuous heart rate measurement option 908 is detected, the electronic device may enter an interface (not shown in the figure) for enabling the continuous heart rate measurement function, and a continuous heart rate monitoring function of the smart band may be enabled by using a function button.

It should be noted that the foregoing interfaces are merely schematic diagrams. In actual application, each interface may include more or less content, or may include more or fewer interfaces. This is not limited in this application.

After the continuous heart rate monitoring function of the electronic device is enabled, the electronic device starts to establish a heart rate baseline of the user. Optionally, the electronic device may further remind the user that establishment of the heart rate baseline has started currently.

Optionally, a motion status of the user may be automatically identified by the electronic device. Alternatively, the motion status may be selected by the user. For example, various types of motion statuses are preset in the electronic device, and the electronic device determines the motion status of the user in response to an operation of selecting the motion status by the user.

Optionally, the obtained heart rate values of the user in different motion statuses meet a preset condition. The preset condition may include preset duration, a preset quantity, and the like. Specifically, that the obtained heart rate values of the user in different motion statuses meet a preset condition may mean that duration of an obtained heart rate value meets the preset duration (for example, one day, two days, or three days), and a quantity of obtained heart rate values meets the preset quantity (for example, 10 to 20). The quantity of heart rate values may be a total quantity of heart rate values (that is, a sum of quantities of heart rate values in all motion statuses), a quantity of heart rate values in each motion status, or the like. This is not limited in this application.

Optionally, the preset condition may further include a confidence requirement, and the confidence requirement is a requirement for a confidence of a heart rate value. When obtaining a heart rate value of the user in a motion status, the electronic device determines whether a confidence of the heart rate value meets a requirement, that is, whether the heart rate value is reliable. For example, it is assumed that the electronic device obtains a heart rate value of 60 when the user is in a fast-running state. However, a heart rate of a human body in a static state ranges from 60 to 200. In this case, the obtained heart rate value may be unreliable.

Optionally, the confidence of the heart rate value may be determined based on one or more factors in quality (for example, PPG signal quality) of a signal used to measure a heart rate of the user and the current motion status of the user. For example, if the confidence requirement is greater than 80%, and a confidence of a heart rate value is 95%, it indicates that the confidence of the heart rate value meets the requirement. Alternatively, if the confidence requirement is greater than 3, and a confidence of a heart rate value is 5, it indicates that the confidence of the heart rate value also meets the requirement. In other words, a manner of representing the confidence of the heart rate value is not limited in this application, and a manner of calculating the confidence of the heart rate value is also not limited in this application.

In addition, to further determine whether the heart rate value is reliable, the electronic device may further perform analysis with reference to an empirical value.

S802: The electronic device establishes a target mapping relationship based on the different motion statuses and the heart rate values.

The electronic device may collect statistics on a heart rate value that is of the user and that is obtained in each motion status, and then determine a heart rate baseline corresponding to each motion status, where the heart rate baseline indicates a heart rate status of the user in the motion status. Further, the electronic device establishes a mapping relationship between different motion statuses and heart rate baselines of the user. As shown in FIG. 10, for the user, a heart rate baseline corresponding to a low activity amount state may be a heart rate value corresponding to 1001, a heart rate baseline corresponding to a medium activity amount state may be a heart rate value corresponding to 1002, and a heart rate baseline corresponding to a sleep state may be a heart rate value corresponding to 1003. For example, the heart rate baseline corresponding to each motion status may be based on statistical data such as an average value, a median, a mode, and a middle value of all heart rate values obtained in the motion status, or may be based on a value range determined based on all heart rate values obtained in the motion status.

It should be noted that the mapping relationship between each motion status and the heart rate baseline may be established simultaneously, or may be established successively. This is not limited in this application.

Optionally, after establishing the target mapping relationship, the electronic device may further remind the user that the baseline is successfully established.

Optionally, after the target mapping relationship is established, the target mapping relationship is associated with account information of a currently logged-in target application. For example, after the electronic device detects an operation of enabling the continuous heart rate monitoring function by the user, the electronic device may obtain the account information of the currently logged-in target application. Subsequently, after the mapping relationship is successfully established, the account information of the currently logged-in target application may be associated with the mapping relationship.

The foregoing mainly describes the solutions provided in embodiments of this application from a perspective of a method. It may be understood that, to implement the foregoing functions, the electronic device includes a corresponding hardware structure and/or software module for performing each of the functions. With reference to the units and algorithm steps described in embodiments disclosed in this application, embodiments of this application can be implemented in a form of hardware or a combination of hardware and computer software. Whether a function is performed by hardware or hardware driven by a computer depends on a particular application and a design constraint condition that are of a technical solution. A person skilled in the art may use different methods to implement the described functions for each particular application, but it should not be considered that the implementation falls beyond the scope of the technical solutions in embodiments of this application.

In embodiments of this application, the electronic device may be divided into functional modules based on the foregoing method examples. For example, each functional module may be obtained through division for each corresponding function, or two or more functions may be integrated into one processing unit. The integrated unit may be implemented in a form of hardware, or may be implemented in a form of a software functional module. It should be noted that, in embodiments of this application, division into the units is an example, and is merely a logical function division. In an actual implementation, another division manner may be used.

FIG. 11 is a schematic diagram of a structure of an electronic device according to an embodiment of this application. An electronic device 1100 may be configured to implement the methods recorded in the foregoing method embodiments. For example, the electronic device 100 may specifically include an obtaining unit 1101 and a processing unit 1102.

The obtaining unit 1101 is configured to support the electronic device 1100 in performing step S701 in FIG. 7; and/or the obtaining unit 1101 is configured to support the electronic device 1100 in performing step S801 in FIG. 8; and/or the obtaining unit 1101 is further configured to support the electronic device 1100 in performing another step performed by the electronic device in embodiments of this application.

The processing unit 1102 is configured to support the electronic device 1100 in performing steps S702 to S705 and S707 in FIG. 7; and/or the processing unit 1102 is configured to support the electronic device 1100 in performing step S802 in FIG. 8; and/or the processing unit 1102 is further configured to support the electronic device 1100 in performing another step performed by the electronic device in embodiments of this application.

Optionally, the electronic device 1100 shown in FIG. 11 may further include a display unit 1103. The display unit 1103 is configured to support the electronic device 1100 in performing step S706 in FIG. 7; and/or the display unit 1103 is further configured to support the electronic device 1100 in performing another step performed by the electronic device in embodiments of this application.

Optionally, the electronic device 1100 shown in FIG. 11 may further include a communication unit (not shown in FIG. 11). The communication unit is configured to support the electronic device 1100 in performing a step of communication between the electronic device and another electronic device in embodiments of this application.

Optionally, the electronic device 1100 shown in FIG. 11 may further include a storage unit (not shown in FIG. 11), and the storage unit stores a program or instructions. When the processing unit 1102 executes the program or the instructions, the electronic device 1100 shown in FIG. 11 may perform the methods shown in FIG. 7 and FIG. 8.

For technical effects of the electronic device 1100 shown in FIG. 11, refer to technical effects of the methods shown in FIG. 7 and FIG. 8. Details are not described herein again. The processing unit 1102 in the electronic device 1100 shown in FIG. 11 may be implemented by a processor or a processor-related circuit assembly, and may be a processor or a processing module. The communication unit may be implemented by a transceiver or a transceiver-related circuit assembly, and may be a transceiver or a transceiver module. The display unit 1103 may be implemented by a display-related assembly.

An embodiment of this application further provides a chip system. As shown in FIG. 12, the chip system includes at least one processor 1201 and at least one interface circuit 1202. The processor 1201 and the interface circuit 1202 may be connected to each other through a line. For example, the interface circuit 1202 may be configured to receive a signal from another apparatus. For another example, the interface circuit 1202 may be configured to send a signal to another apparatus (for example, the processor 1201). For example, the interface circuit 1202 may read instructions stored in a memory, and send the instructions to the processor 1201. When the instructions are executed by the processor 1201, an electronic device may be enabled to perform the steps performed by the electronic device in the foregoing embodiments. Certainly, the chip system may further include another discrete device. This is not specifically limited in this embodiment of this application.

Optionally, there may be one or more processors in the chip system. The processor may be implemented by using hardware, or may be implemented by using software. When the processor is implemented by using the hardware, the processor may be a logic circuit, an integrated circuit, or the like. When the processor is implemented by using the software, the processor may be a general-purpose processor, and is implemented by reading software code stored in the memory.

Optionally, there may be one or more memories in the chip system. The memory may be integrated with the processor, or may be disposed separately from the processor. This is not limited in this application. For example, the memory may be a non-transitory processor, for example, a read-only memory ROM. The memory and the processor may be integrated into a same chip, or may be separately disposed on different chips. A type of the memory and a manner of disposing the memory and the processor are not specifically limited in this application.

The chip system may be a field programmable gate array (field programmable gate array, FPGA), an application-specific integrated circuit (application-specific integrated circuit, ASIC), a system on chip (system on chip, SoC), a central processing unit (central processing unit, CPU), a network processor (network processor, NP), a digital signal processor (digital signal processor, DSP), a micro controller unit (micro controller unit, MCU), a programmable logic device (programmable logic device, PLD), or another integrated chip.

It should be understood that the steps in the foregoing method embodiments may be completed by using an integrated logic circuit of hardware in the processor or instructions in a form of software. The steps of the method disclosed with reference to embodiments of this application may be directly performed by a hardware processor, or may be performed through a combination of hardware and software modules in the processor.

An embodiment further provides a computer storage medium. The computer storage medium stores computer instructions. When the computer instructions are run on an electronic device, the electronic device is enabled to perform the methods in the foregoing method embodiments.

An embodiment of this application provides a computer program product, where the computer program product includes a computer program or instructions. When the computer program or the instructions is/are run on a computer, the computer is enabled to perform the methods in the foregoing method embodiments.

In addition, an embodiment of this application further provides an apparatus. The apparatus may be specifically a chip, an assembly, or a module. The apparatus may include a processor and a memory that are connected. The memory is configured to store computer-executable instructions. When the apparatus runs, the processor may execute the computer-executable instructions stored in the memory, to enable the apparatus to perform the methods in the foregoing method embodiments.

The electronic device, the computer storage medium, the computer program product, or the chip provided in embodiments is configured to perform the corresponding method provided above. Therefore, for beneficial effects that can be achieved, refer to beneficial effects in the corresponding method provided above. Details are not described herein again.

Based on the foregoing descriptions of the implementations, a person skilled in the art may understand that for the purpose of convenient and brief description, division into the foregoing functional modules is merely used as an example for illustration. During actual application, the foregoing functions can be allocated to different functional modules for implementation based on a requirement, that is, an inner structure of an apparatus is divided into different functional modules to implement all or some of the functions described above.

In several embodiments provided in this application, it should be understood that the disclosed apparatus and method may be implemented in another manner. The embodiments may be mutually combined or referenced when there is no conflict. The described apparatus embodiment is merely an example. For example, division of the modules or units is merely logical function division and may be other division in an actual implementation. For example, a plurality of units or components may be combined or integrated into another apparatus, or some features may be ignored or not performed. In addition, the displayed or discussed mutual couplings or direct couplings or communication connections may be implemented through some interfaces. The indirect couplings or communication connections between the apparatuses or units may be implemented in electronic, mechanical, or other forms.

The units described as separate parts may or may not be physically separate, and parts displayed as units may be one or more physical units, may be located in one place, or may be distributed on different places. Some or all of the units may be selected based on an actual requirement to achieve the objectives of the solutions of embodiments.

In addition, functional units in embodiments of this application may be integrated into one processing unit, each of the units may exist alone physically, or two or more units may be integrated into one unit. The integrated unit may be implemented in a form of hardware, or may be implemented in a form of a software functional unit.

When the integrated unit is implemented in a form of a software function unit and sold or used as an independent product, the integrated unit may be stored in a readable storage medium. Based on such an understanding, the technical solutions of embodiments of this application essentially, or the part contributing to the conventional technology, or all or some of the technical solutions may be implemented in a form of a software product. The software product is stored in a storage medium and includes several instructions for instructing a device (which may be a single-chip microcomputer, a chip, or the like) or a processor (processor) to perform all or some of the steps of the methods in embodiments of this application. The foregoing storage medium includes any medium that can store program code, for example, a USB flash drive, a removable hard disk, a read-only memory (read-only memory, ROM), a random access memory (random access memory, RAM), a magnetic disk, or an optical disc.

The foregoing descriptions are merely specific implementations of this application, but are not intended to limit the protection scope of this application. Any variation or replacement readily figured out by a person skilled in the art within the technical scope disclosed in this application shall fall within the protection scope of this application. Therefore, the protection scope of this application shall be subject to the protection scope of the claims.

## Claims

1. A body temperature measurement method, applied to an electronic device, wherein the method comprises:
obtaining a target parameter, wherein the target parameter comprises motion data of a user and a current heart rate value of the user, and the motion data is used to determine a current motion status of the user;
determining a body temperature of the user based on the current motion status of the user and the current heart rate value of the user; and
reminding the user of the body temperature.

2. The method according to claim 1, wherein the determining a body temperature of the user based on the current motion status of the user and the current heart rate value of the user comprises:
determining a target heart rate baseline based on the current motion status of the user, wherein the target heart rate baseline indicates a heart rate status of the user in the current motion status; and
determining the body temperature of the user based on the current heart rate value of the user and the target heart rate baseline.

3. The method according to claim 2, wherein before the determining a target heart rate baseline based on the current motion status of the user, the method further comprises:
obtaining account information of the user; and
obtaining a mapping relationship between different motion statuses and heart rate baselines of the user based on the account information; and
the determining a target heart rate baseline based on the current motion status of the user comprises:
determining the target heart rate baseline based on the current motion status of the user and the mapping relationship.

4. The method according to claim 2 or 3, wherein before the determining a target heart rate baseline based on the current motion status of the user, the method further comprises:
if no mapping relationship between different motion statuses and heart rate baselines of the user is obtained, reminding the user to establish the mapping relationship.

5. The method according to any one of claims 2 to 4, wherein if no mapping relationship between different motion statuses and heart rate baselines of the user is obtained, the method further comprises:
obtaining heart rate values of the user in the different motion statuses; and
establishing the mapping relationship based on the different motion statuses and the heart rate values.

6. The method according to claim 5, wherein the obtained heart rate values of the user in the different motion statuses meet a requirement for a confidence.

7. The method according to claim 6, wherein the confidence is determined based on one or more factors in quality of a signal used to measure a heart rate of the user and the current motion status of the user.

8. The method according to any one of claims 1 to 7, wherein the target parameter further comprises one or more of a skin temperature of a target part of the user and a current ambient temperature, and the method further comprises:
determining the body temperature of the user based on the current motion status of the user, the current heart rate value of the user, the skin temperature of the target part of the user, and/or the current ambient temperature.

9. The method according to any one of claims 3 to 8, wherein the different motion statuses comprise a sleep state and a non-sleep state.

10. The method according to claim 9, wherein the non-sleep state comprises a low activity amount state, a medium activity amount state, and a high activity amount state; and
the low activity amount state is a state in which an activity amount of the user is less than a first threshold, the medium activity amount state is a state in which an activity amount of the user is greater than or equal to the first threshold and less than or equal to a second threshold, and the high activity amount state is a state in which an activity amount of the user is greater than the second threshold.

11. The method according to any one of claims 3 to 8, wherein the different motion statuses comprise one or more of the following: a slow-walking state, a fast-walking state, a jogging state, a fast-running state, a riding state, and a rope skipping state.

12. The method according to any one of claims 1 to 11, wherein the reminding the user of the body temperature comprises:
displaying the body temperature; and/or
broadcasting the body temperature by voice.

13. An electronic device, comprising a processor and a memory, wherein the memory is coupled to the processor, the memory is configured to store computer program code, the computer program code comprises computer instructions, and when the processor reads the computer instructions from the memory, the electronic device is enabled to perform the method according to any one of claims 1 to 12.

14. A computer-readable storage medium, wherein the computer-readable storage medium stores a computer program or instructions, and when the computer program or the instructions is/are run on a computer, the computer is enabled to perform the method according to any one of claims 1 to 12.

15. A computer program product, wherein the computer program product comprises a computer program or instructions, and when the computer program or the instructions is/are run on a computer, the computer is enabled to perform the method according to any one of claims 1 to 12.
